# EUROPEAN PATENT APPLICATION

(11) **EP 3 421 052 A1**
(43) Date of publication of application: **02.01.2019**
(21) Application number: 17001088.8
(22) Date of filing: 26.06.2017
(51) Int. Cl.: A61K 47/69, A61K 47/64, A61K 41/00, C12N 7/00

(54) **A CONJUGATE COMPLEX FACILITATING THE TRANSPORT OF A CARGO THROUGH A MEDIUM**

(71) Applicant: Baden-Württemberg Stiftung gGmbH, 70174 Stuttgart (DE)
(72) Inventor: Rothenstein, Dirk, 70190 Stuttgart (DE); Bill, Joachim, 75365 Calw (DE); Fischer, Peer, 79100 Freiburg (DE)
(74) Representative: Müller-Boré & Partner Patentanwälte PartG mbB

(57) **Abstract**

The present invention relates to conjugate complexes, comprising at least one biological entity, at least one cargo moiety, and at least one effector moiety that is capable of converting, degrading, and/or modifying a given medium, wherein the at least one cargo moiety and the at least one effector moiety are directly or indirectly coupled to the biological entity. The present invention further relates to uses thereof and methods for facilitating the transport of a cargo moiety through a given medium.

## Description

The present invention relates to conjugate complexes, comprising at least one biological entity, at least one cargo moiety, and at least one effector moiety that is capable of converting, degrading, and/or modifying a given medium, wherein the at least one cargo moiety and the at least one effector moiety are directly or indirectly coupled to the biological entity. The present invention further relates to uses thereof and methods for facilitating the transport of a cargo moiety through a given medium.

The human body has numerous mechanisms that protect it against the invasion of pathogens. These defenses include protective layers such as e.g. mucosal layers that line all wet epithelial surfaces, including the airway system and lungs, the gastrointestinal tract, and the urogenital tract. Mucus barriers not only make it difficult for microorganisms to reach and penetrate the host's tissues but also hinder the delivery of other substances such as potential pharmaceutical carriers, while allowing nutrients and other beneficial substances to pass freely. The stomach and gastrointestinal tract pose a particular challenge in this context, because here mucus layer presents a major obstacle for various drug delivery applications. At the same time, most pharmaceuticals on the market are designed for oral administration; hence, efficient approaches for drug uptake in the gastrointestinal tract are of significant medical importance.

Accordingly, the technical problem underlying the present invention is to provide carriers that can overcome protective layers in the human body, such as the mucus lining in the stomach. Other biological fluids and gel-like media such as the vitreous humor of the eye also present barriers to the free diffusion of given cargo moieties. These barriers therefore hinder the delivery and targeted application of e.g. pharmaceutical agents that are administered in particulate form.

The solution to the above technical problem is achieved by the embodiments characterized in the claims.

In particular, in a first aspect, the present invention relates to a conjugate complex comprising:
(a) at least one biological entity;
(b) at least one cargo moiety; and
(c) at least one effector moiety that is capable of converting, degrading and/or modifying a given medium;
wherein the at least one cargo moiety and the at least one effector moiety are directly or indirectly coupled to the biological entity.

The term "conjugate complex" as used herein refers to a construct comprising the above elements which are coupled, *i.e.,* conjugated, to each other via covalent or non-covalent interactions.

The biological entity which forms the central part of the conjugate complex of the present invention is preferably a self-assembling biological entity comprising proteinaceous coat. In preferred embodiments, the biological entity is a virus, including a bacteriophage. In further embodiments, the biological entity comprises at least two functionalities for coupling at least one effector moiety. Such biological entities include viral nanoparticles (VNP), virus like particles (VLP), bacteriophages, proteinaceous assemblies and modifications thereof. The conjugate complex of the present invention can comprise one or more biological entities, e.g. 1, 2, 3, 4, 5, 6, 7, 8, 9, 10, or more biological entities, up to several thousand biological moieties or more, wherein in particular embodiments, only one biological entity is present. Further, the conjugate complex of the present invention can comprise different biological entities. In specific embodiments, the biological entity is genetically, chemically and/or biochemically modified. Suitable modifications include the permanent or transient binding of two or more components with each other by peptides, His-tag, protein tags, antibodies, as well as the covalent coupling with NH₂, COOH, SH, or OH groups.

The cargo moiety which is part of the conjugate complex of the present invention can be any cargo moiety transport of which through a given medium is desired. In preferred embodiments, the cargo moiety is selected from the group consisting of organic or inorganic molecules, nucleic acids, peptides, proteins, organic or inorganic micro- or nanoparticles, magnetic micro- or nanoparticles, piezoelectric micro- or nanoparticles, hydrophilic micro- or nanoparticles, hydrophobic micro-or nanoparticles, biological entities as defined herein, or combinations thereof. In preferred embodiments, the cargo moiety is a molecule or a pharmaceutical agent, e.g. a particulate pharmaceutical agent. The conjugate complex of the present invention can comprise one or more cargo moieties, e.g. 1, 2, 3, 4, 5, 6, 7, 8, 9, 10, or more cargo moieties, up to several thousand or more cargo moieties. Further, the conjugate complex of the present invention can comprise different cargo moieties, e.g. two or more different pharmaceutical agents, or a pharmaceutical agent and a further cargo moiety such as e.g. a magnetic particle.

The effector moiety which is part of the conjugate complex of the present invention can be any moiety that is capable of converting, degrading and/or modifying a given medium. Suitable effector moieties are not particularly limited and are known in the art for different given media. In particular embodiments, the medium is a tissue, and organ, or protective layer of the human or animal body, wherein the protective layer is e.g. selected from the group consisting of biological fluids and biological gel-like media, such as mucus linings, mucin, the vitreous humor of the eye, lymphatic fluid (lymph), linings of the lymphatic system, cerebrospinal fluid, and myelin in the brain. In preferred embodiments, the effector moiety is selected from the group consisting of enzymes, e.g. ureases, hyaluronidases, proteases and collagenases, peptides, catalysts, and functional chemical groups, and biological entities as defined herein, wherein ureases are a particular example. The conjugate complex of the present invention can comprise one or more effector moieties, e.g. 1, 2, 3, 4, 5, 6, 7, 8, 9, 10, or more effector moieties. Further, the conjugate complex of the present invention can comprise different effector moieties, e.g. a urease and a collagenase, or a ureases and a specific catalyst.

The conjugate complex of the present invention can comprise further moieties, e.g. moieties providing functions such as sensing functions, catalytic activities, and tracking and/or marking functions, e.g. via suitable dyes.

In particular embodiments, the conjugate complex of the present invention is self-assembling, *i.e*., it forms spontaneously when all components of the conjugate complex are brought together in a suitable solution. Alternatively, the conjugate complex of the present invention can be assembled by chemical, biochemical, biological and/or physical means known in the art.

Coupling of the cargo moiety, effector moiety and optional further moieties to the biological entity can be via covalent or non-covalent interactions, such as electrostatic interactions, π-effects, van der Waals forces, hydrophobic effects, and entropic effects. Suitable coupling strategies are not particularly limited and are known in the art. They include for example the use of suitable linkers, e.g. bifunctional linkers, for covalent coupling, the use of peptide or protein tags, such as His-tag binding to nickel or cobalt chelate, or StrepTag binding to streptavidin, and the use of antibodies, antibody fragments, antibody mimetics, aptamers or ionic coupling.

In particular embodiments, the conjugate complex of the present invention is for use in medicine. In further embodiments, the conjugate complex of the present invention is for use in the delivery of pharmaceutical agents. Further, the conjugate complex of the present invention can be used in nanotechnology and technical applications.

In a second aspect, the present invention relates to a method for facilitating the transport of a cargo moiety through a medium, comprising the step of contacting said medium with a conjugate complex according to the present invention.

In this aspect, all relevant definitions, limitations and embodiments as defined for the first aspect of the present invention apply in an analogous manner.

The method of the present invention can be performed *in vivo* or *in vitro.* In particular embodiments, the method is performed *in vitro, i.e.,* it is not performed on the human or animal body.

In specific embodiments, the actual transport of the cargo moiety through a given medium is by diffusion. In other specific embodiments, a cargo moiety is a magnetic particle and the transport is effected by application of a magnetic field, or a cargo moiety is a light-interacting particle and the transport is effected by application of a light field, or a cargo moiety allows for self-propulsion by means of chemical reactions.

In a final third aspect, the present invention relates to the use of the conjugate complex of the present invention for facilitating the transport of a cargo moiety through a medium.

In this aspect, all relevant definitions, limitations and embodiments as defined for the first and second aspect of the present invention apply in an analogous manner.

As used herein, the term "about" is intended to be a modifier of ± 10% of the specified value. As an example, the term "about 5%" is intended to encompass the range of 4.5 to 5.5%.

The terms "comprising/comprises", "consisting of/consists of', and "consisting essentially of/consists essentially of" are used herein in an interchangeable manner, *i.e.,* each of said terms can expressly be exchanged against one of the other two terms.

The present invention relates to conjugate complexes facilitating the transport of a cargo moiety of interest or of many cargo moieties of interest, e.g. a pharmaceutical agent or a plurality of pharmaceutical agents, through a given medium, e.g. a protective layer of the human body. This can be advantageously used e.g. to increase the efficacy of a pharmaceutical agent. The mechanism underlying the present invention is based on the directed modification of the microenvironment by an effector molecule that is part of the conjugate complex.

This microenvironment modification increases the mobility of the complex in the respective medium. Actual movement of the conjugate complex can be effected by diffusion, or can be effected in a directed manner using suitable cargo moieties such as e.g. magnetic particles and applying a magnetic field.
The figures show:
Figure 1:
   Schematic representation of a conjugate complex of the present invention comprising its single components, the biological entity, the cargo moiety and the effector moiety, and the coupling strategy.
Figure 2:
   Schematic representation of the mode of action of a conjugate complex of the present invention. The a conjugate complex is moved actively or passively. Effector moieties are coupled on the biological entity for converting, degrading and/or modifying a medium.

The present invention will be further illustrated in the following examples without being limited thereto.

### Examples

### Material and Methods:

*DNA primers.*
p3HT-for 
5´ AGTGGTACCTTTCTATTCTCACTCTCATCATCACCATCACCACCTGGTTCCGCGTG
GATCCTCGGCCGAAA 3´ (SEQ ID NO: 1)
p3HT-rev 
5´ TTTCGGCCGAGGATCCAGCGGAACCAGGTGGTGATGGTGATGATGAGAGTGAGAA
TAGAAAGGTACCACT 3´ (SEQ ID NO: 2)
p7HXa_minus 
5´ TCTGCGCCGCTAGCATTGATGGACGTATGGAGCAGGTCGCGGATTTCGACACAATT
TATC 3´ (SEQ ID NO: 3)
p7HXa_plus 
5´ AATTGTGCTAGCGTGGTGATGGTGATGATGCATGTTACTTAGCCGGAACGAGGCGC
AGAC 3´ (SEQ ID NO: 4)
p9HXa_minus 
5´ TGGTATGCTAGCATTGATGGACGTATGAGTGTTTTAGTGTATTCTTTTGCCTCTTT
CGTT 3´ (SEQ ID NO: 5)
p9HXa_plus

5´ ACGAAAGAGCTAGCGTGGTGATGGTGATGATGCATCTTTGACCCCCAGCGATTATA
CCAA 3´ (SEQ ID NO: 6)

### Example 1:

### Genetic modifications of bacteriophages.

The His-tag fusion proteins with the minor coat proteins p3, p7, and p9, respectively, of M13 bacteriophages and fd Y21M bacteriophages were established by genetic manipulations.

**p3-His-tag:** Single stranded DNA primers p3HT-for and p3HT-rev were hybridized and digested with the restriction enzymes Bam HI and Kpn I, 1 hour at 37°C, and gel purified. This DNA fragment is referred to as "insert" hereinafter. M13KE bacteriophage DNA was digested with restriction enzymes Hind III and Kpn I, 1 hour at 37°C, and gel purified. This DNA fragment is referred to as "vector" hereinafter. Appropriate amounts of insert and vector were ligated by T4 DNA ligase and transformed into *Escherichia coli* ER2738 cells. Bacteriophage clones expressing the His-tag were determined by DNA sequencing.

**p7-His-tag** / **p9**-**His**-**tag:** His-tags were introduced by reverse polymerase chain reaction (PCR) with abutting primers introducing an additional Nhe I restriction site. M13KE bacteriophage DNA was used as template for the introduction of the His-tag and Nhe I restriction site by reverse PCR with primers p9HXa_minus / p9HXa_plus and p7HXa_minus / p7HXa_plus, respectively. PCR products were digested with Nhe I restriction enzyme and ligated with T4 DNA ligase. *Escherichia coli* ER2738 cells were transformed with one of the constructs. Bacteriophage clones were identified by DNA sequencing. The fd Y21 M bacteriophages were genetically modified in an analogous manner.

Bacteriophages expressing His-tag as fusion to any minor coat protein couple to Ni-NTA surface coated magnetic beads.

### Example 2:

### Covalent coupling of urease to M13 bacteriophage.

Specific urease was coupled to bacteriophage by Sulfo-SIAB bifunctional linker. 50 µL His-tagged bacteriophage-magnetic beads were incubated with 10 µL Sulfo-SIAB in 440 µL phosphate buffer for one hour at 20 °C. Excessive Sulfo-SIAB was eliminated by washing with phosphate buffer. Bacteriophage-magnetic beads were resuspended in 500 µL phosphate buffer and mixed with 200 µL (0.05 mg/µL) urease in phosphate buffer and incubated one hour, 20 °C in the dark. Excessive urease was eliminated by washing steps with phosphate buffer.

SEQUENCE LISTING

<110>  Baden-Württemberg Stiftung gGmbH

<120>  A conjugate complex facilitating the transport of a cargo through
       a medium

<130>  B 3471EU - kl

<160>  6

<170>  PatentIn version 3.5

<210>  1
<211>  71
<212>  DNA
<213>  Artificial sequence

<220>
<223>  Primer sequence

<400>  1
agtggtacct ttctattctc actctcatca tcaccatcac cacctggttc cgcgtggatc      60

ctcggccgaa a                                                           71


<210>  2
<211>  71
<212>  DNA
<213>  Artificial sequence

<220>
<223>  Primer sequence

<400>  2
tttcggccga ggatccagcg ggaaccaggt ggtgatggtg atgatgagag tgagaataga      60

aaggtaccac t                                                           71


<210>  3
<211>  60
<212>  DNA
<213>  Artificial sequence

<220>
<223>  Primer sequence

<400>  3
tctgcgccgc tagcattgat ggacgtatgg agcaggtcgc ggatttcgac acaatttatc      60


<210>  4
<211>  60
<212>  DNA
<213>  Artificial sequence

<220>
<223>  Primer sequence

<400>  4
aattgtgcta gcgtggtgat ggtgatgatg catgttactt agccggaacg aggcgcagac      60

<210>  5
<211>  60
<212>  DNA
<213>  Artificial sequence

<220>
<223>  Primer sequence

<400>  5
tggtatgcta gcattgatgg acgtatgagt gttttagtgt attcttttgc ctctttcgtt        60


<210>  6
<211>  60
<212>  DNA
<213>  Artificial sequence

<220>
<223>  Primer sequence

<400>  6
acgaaagagc tagcgtggtg atggtgatga tgcatctttg acccccagcg attataccaa        60

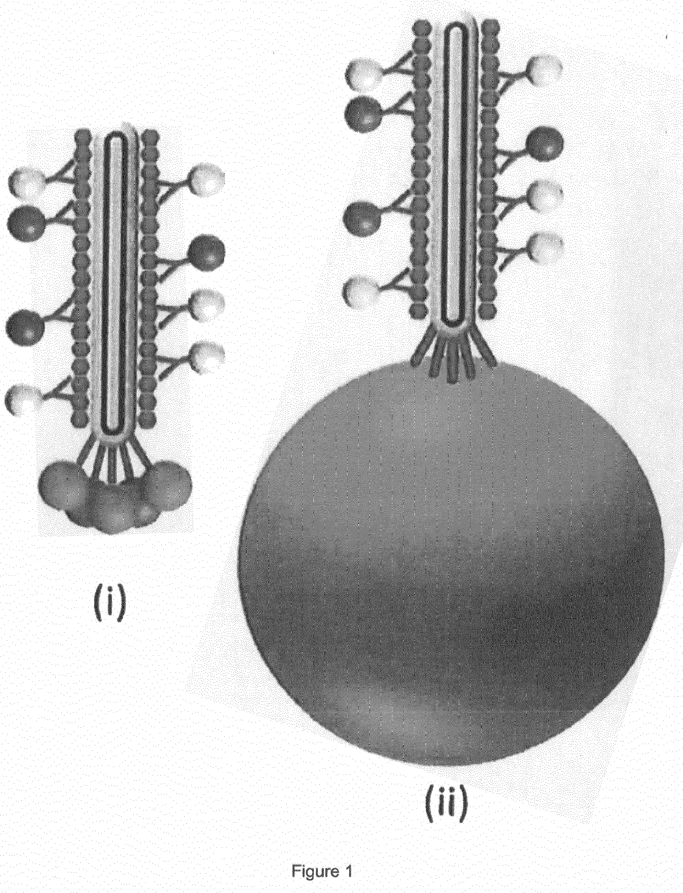

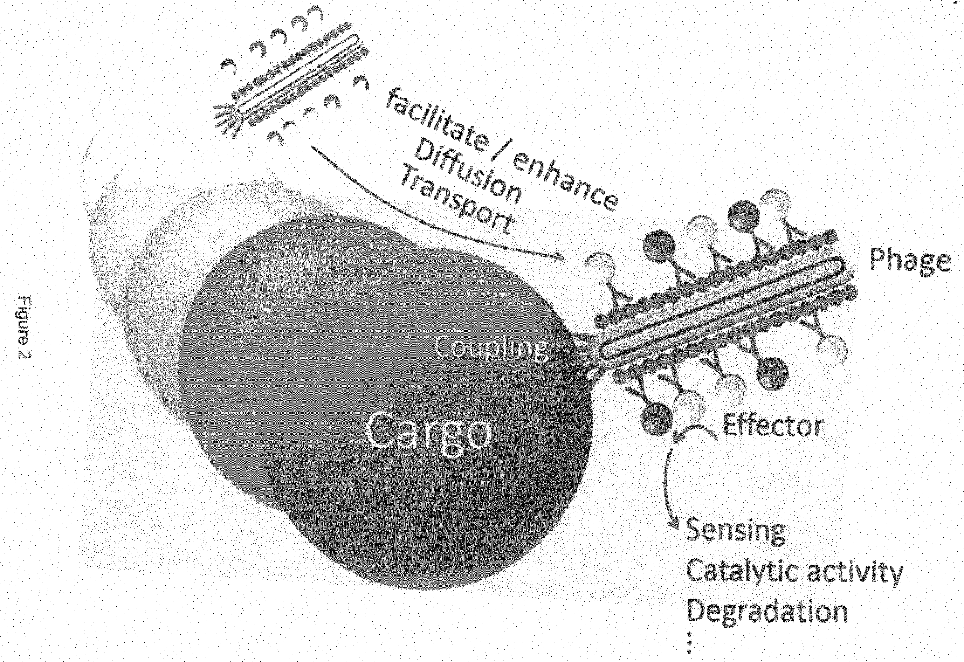

## Claims

1. A conjugate complex comprising:
(a) at least one biological entity;
(b) at least one cargo moiety; and
(c) at least one effector moiety that is capable of converting, degrading and/or modifying a given medium;
wherein the at least one cargo moiety and the at least one effector moiety are directly or indirectly coupled to the biological entity.

2. The conjugate complex of claim 1, wherein the biological entity is selected from the group consisting of viral nanoparticles (VNP), virus like particles (VLP), and bacteriophages.

3. The conjugate complex of claim 1 or claim 2, wherein the cargo moiety is selected from the group consisting of organic or inorganic molecules, nucleic acids, peptides, proteins, organic or inorganic micro- or nanoparticles, magnetic micro- or nanoparticles, piezoelectric micro- or nanoparticles, hydrophilic micro- or nanoparticles, hydrophobic micro- or nanoparticles, and biological entities.

4. The conjugate complex of any one of claims 1 to 3, wherein the cargo moiety is a pharmaceutical agent.

5. The conjugate complex of any one of claims 1 to 4, wherein the medium is a tissue, an organ, or a protective layer in the human or animal body, wherein the protective layer is selected from the group consisting of biological fluids and biological gel-like media.

6. The conjugate complex of claim 5, wherein the protective layer is selected from the group consisting of mucus linings, and the vitreous humor of the eye.

7. The conjugate complex of any one of claims 1 to 6, wherein the effector moiety is selected from the group consisting of enzymes, peptides, catalysts, functional chemical groups, and biological entities.

8. The conjugate complex of claim 7, wherein the effector moiety is an enzyme selected from the group consisting of ureases, hyaluronidases, proteases, peptidases, and collagenases.

9. The conjugate complex of any one of claims 1 to 8, wherein the conjugate complex is self-assembling.

10. The conjugate complex of any one of claims 1 to 9, wherein coupling of the at least one cargo moiety and/or the at least one effector moiety to the biological entity is via covalent binding or via non-covalent interactions.

11. The conjugate complex of any one of claims 1 to 10 for use in medicine.

12. A method for facilitating the transport of a cargo moiety through a medium, comprising the step of contacting said medium with the conjugate complex of any one of claims 1 to 10.

13. The method of claim 12, wherein the transport is effected by diffusion.

14. The method of claim 12, wherein a cargo moiety is a magnetic particle and the transport is effected by application of a magnetic field, or a cargo moiety is a light-interacting particle and the transport is effected by application of a light field, or a cargo moiety allows for self-propulsion by means of chemical reactions.

15. Use of the conjugate complex of any one of claims 1 to 10 for facilitating the transport of a cargo moiety through a medium.
